Europäisches Patentamt

(19) European Patent Office

Office européen des brevets

(11) Publication number: **0 129 425**
**B1**

# EUROPEAN PATENT SPECIFICATION

(12)

(45) Date of publication of patent specification: **23.08.89**

(21) Application number: **84304055.1**

(22) Date of filing: **15.06.84**

(51) Int. Cl.⁴: **G 01 N 33/49**

(54) Method and apparatus for measuring haemostasis and thrombolysis.

(30) Priority: **15.06.83 GB 8316332**

(43) Date of publication of application:
**27.12.84 Bulletin 84/52**

(45) Publication of the grant of the patent:
**23.08.89 Bulletin 89/34**

(84) Designated Contracting States:
**DE FR GB IT SE**

(56) References cited:
**GB-A-2 096 329**

**NATURE, vol. 259, January 22, 1976, pages
233-235, Basingstoke, GB; Born et al.:
"Evidence for inhibition of platelet activation in
blood by a drug effect on erythrocytes"
M. Kratzer et al., Haemostasis 15:357-62, 1985**

(73) Proprietor: **Gorog, Peter
20 Grenville Court Lymer Avenue
London SE19 (GB)**

(72) Inventor: **Gorog, Peter
20 Grenville Court Lymer Avenue
London SE19 (GB)**

(74) Representative: **Raynor, John et al
W.H. Beck, Greener & Co 7 Stone Buildings
Lincoln's Inn
London WC2A 3SZ (GB)**

Courier Press, Leamington Spa, England.

# Description

This invention relates generally to methods and apparatus for measuring haemostasis, that is to say cessation of bleeding, and to certain other methods and apparatus useful in connection with the determination of haemostasis.

There is an urgent need to provide a simple technique for detecting haemorrhagic disorders, in particular so that the risk of bleeding after surgery can be assessed. At present, a number of different time-consuming tests must be performed in a specialised haemotological diagnostic laboratory, to assess the risk of bleeding in a particular patient. Not only is the known procedure time-consuming, and thus unsuitable for use in emergency, but also the results are not always consistent.

A method of *in vitro* analysis of haemostasis time was suggested by Blakley *et al* (J. Lab. Clin. Med 89: 1306—1314 (1976). In this method, a roller pump is made to circulate blood through a plastic tube, in which has been provided a hole drilled to simulate damage to a blood vessel. It is found that substantial damage results to the red cells of the blood because of the use of the peristaltic pump, the pumping action of which relies on the squashing of a flexible tube, and consequently squashes the red cells contained in the tube. In the device disclosed in the reference, the problem is exacerbated by the fact that the blood is recirculated, and thus passes through the peristaltic pump many times.

Such damage results in the release of substantial quantities of adenosine diphosphate (ADP) which renders the measurements obtained completely non-representative of the *in vivo* situation. Furthermore, it is found that it is impossible to obtain satisfactory reproducibility in the holes drilled in the plastic tube.

For these reasons, the device disclosed in the reference has been unsatisfactory, and the commercial embodiment which resulted from it has been completely unsuccessful in the market place.

An article by G. V. Born (Nature *259*: 233—235, 1976) discloses a generall similar device.

U.K. Patent Application No. 2096329 also discloses a method for measuring, for example, platelet agglomeration, in which blood is caused to flow through a pre-formed hole in a metal disc. This method is again found to be unsatisfactory, and to bear little relationship to the *in vivo* situation, and to result in non-reproducible measurements.

It is believed that the difficulties with this latter device arise in large part because a substantial amount of platelet aggregation and coagulation has already occurred at the air/blood interface, as the blood first passes through the device.

A later proposal by the same authors (M. Kratzer and G. V. R. Born; Haemostasis 15: 357—62, 1985) which has been utilised in a commercial device in preference to the one disclosed in U.K. Patent Application No. 2096329, involves the drawing of the blood sample through a polycarbonate filter soaked with ADP and collagen. This technique also suffers from the drawbacks noted above of lack of reproducibility and lack of relevance to the real *in vivo* situation.

I have found that far more consistent results can be obtained by causing blood to pass through a tube, and piercing the tube whilst the blood flows therein, using a needle having a substantially parallel-sided shank.

According to a first aspect of the invention therefore, there is provided apparatus for monitoring the haemostasic reaction of a blood sample, which apparatus comprises a container for the blood sample, means for causing the blood sample to flow through a tube, for example a capillary tube, a support for the tube, and a needle having a substantially parallel-sided shank mounted for movement to pierce a hole in a wall, and preferably in both walls of the tube, substantially perpendicular to its surface, and means for monitoring the blood flow through the hole.

The use of such apparatus makes it possible to pierce the hole in the tube whilst the blood is actually flowing therein, and it has been found that this provides much more consistent results.

Apparatus as described above preferably includes an optical system of some kind for viewing the tube under magnification, for example an optical microscope, an optical projection system for projecting an image of the tube onto a screen, or, preferably a closed-circuit television system, to enable the operator to determine the precise positioning of the tube and needle, so that the needle pierces the tube perpendicularly to its surface. The provision of such an optical system also assists the clinician to visualise the bleeding and verify the recorded measurements with visual observation.

According to a further aspect of the invention, there is therefore provided apparatus of the kind described, includes means for observing under magnification the site of piercing of the tube to observe the haemostasis of the blood.

A support for the tube is preferably in the form of a jig adapted to hold the tube in a fixed position, and to support a parallel-sided needle for movement towards and away from the tube. A clearance hole for the needle is preferably provided in the support opposite the needle. Furthermore, a flow-path is preferably provided in the region of the intended hole for a saline solution to wash blood away from the region of the hole as it exits the tube, to facilitate observation by the optical system.

In a further aspect of the invention, there is provided a method for determining the haemostatic reaction of a blood sample, which method comprises causing the blood to flow through the tube of such apparatus under substantially constant pressure, piercing a hole through both walls of the tube whilst the blood is flowing in it with the substantially parallel-sided needle, and measuring the time necessary for blood to flow through the holes to substantially cease. Preferably, the pattern of pressure change in the blood vessel is monitored to give an indication of the nature of the haemostatic reaction. Observation of the shape of the pressure trace as haemostasis takes place can give a useful indication of various haemostatic disorders.

It has also been discovered that the back-pressure applied to the blood flowing in the tube can have a substantial effect on the haemostasis time measured. In particular, it has been found desirable to provide associated with the downstream side of the tube means for providing a constant, but adjustable back-pressure, preferably close to the arterial pressure of approximately 60 mmHg (1 mmHg≙133,322 Pa).

Particularly when such a back-pressure device is utilised, a convenient measure of when the blood flow through the hole has ceased can be provided by monitoring the pressure applied to the blood sample, for example in the blood container.

A further and particularly serious defect of the haemostasis measuring technique suggested by Blakley *et al* is that the pump used causes severe and unquantifiable damage to the blood corpuscles, which tends to render the results obtained irrelevant to the *in vivo* situation or non-reproducible. Any pumping mechanism such as the roller-type peristaltic pump used by Blakley *et al* will cause physical damage to the blood cells, and thus the likelihood of inconsistent results. Even if physical dmage to blood cells cannot be observed, any agitation of this kind is likely to cause the release of ADP, which has an effect on haemostasis.

The susceptibility of blood cells to damage and ADP release is of particular concern in apparatus of this kind, since blood is also subject to settling of the corpuscles therein. Clearly, settling will also have an effect on haemostasis time, and the blood must therefore be kept constantly stirred before it is passed to the measuring tube of the apparatus.

I have now discovered that blood, can be pumped, that is to say caused to flow in a tube, and simultaneously stirred to prevent settling, by displacement with a suitable fluid which is immiscible therewith, and which will not cause an adverse reaction with it.

According to yet a further aspect of the invention therefore, the container for the blood sample may be a columnar container, having an outlet for the blood at the lower end thereof, and means may be provided for causing a displacing fluid, which is not miscible with the blood, for example a liquid hydrocarbon such as paraffin oil, to pass into the lower end of the columnar container, to simultaneously stir the blood, and displace it through the outlet. The displacing fluid can be pumped into the columnar container by any suitable means, for example using a syringe pump.

The diameter of the columnar container is preferably such that the droplets of the immiscible displacing fluid which form in the column are substantial in size in comparison with the column diameter, and thereby have a substantial stirring effect on all the bulk of liquid in the column.

Preferred embodiments of a number of the various aspects of the invention will now be illustrated in detail with reference to the accompanying drawings, in which:

Figure 1 is a schematic diagram of apparatus according to the invention for measuring the rate of haemostasis in a blood sample,

Figure 2 is a perspective view of a jig suitable for use in the apparatus of Figure 1,

Figure 3 is an exploded view of the jig of Figure 2, and

Figure 4 is a pressure trace obtained with the apparatus of Figure 1.

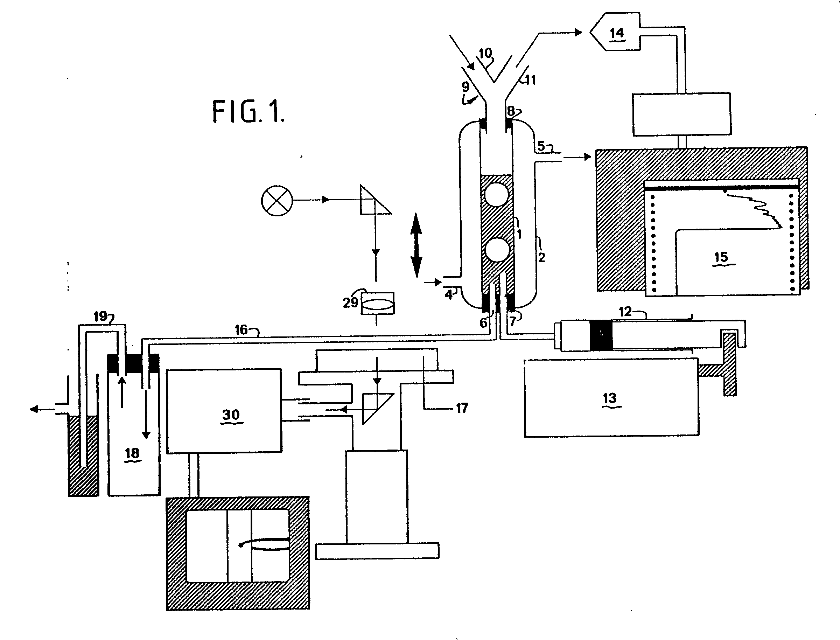

Referring first to Figure 1, apparatus for measuring blood haemostasis comprises a container 1 for the blood provided with a water jacket 2 having a water inlet 4 and a water outlet 5. Water at a constant temperature of 37°C may be passed through the waterjacket. Again, this assists consistency of the results measured. The container 1 has an outlet 6 for blood at its lower end, in the form of a tube received in a rubber bung. The bung also receives an inlet 7 for a displacing fluid, for example paraffin oil. Received in a further bung 8 in the upper end of the container 1 is a Y-tube, having arms 10 and 11.

Arm 10 is used for introducing the blood sample into the container 10. After the blood sample is introduced, an amount of the displacing fluid, paraffin oil, is also introduced, to form a layer on the surface of the blood. The arm 10 is then sealed.

Arm 11 is sealed to the atmosphere, and contains a pressure transducer to enable measurement of the blood flow to be recorded.

In an alternative embodiment, the blood outlet 6 and the fluid inlet 7 may be concentric. The inlet 7 is connected to a conventional syringe pump 12, which may be driven by a conventional motor 13.

The pressure transducer 14 in arm 11 of the Y-tube 9 may be connected to conventional data capture apparatus, which may, for example include a chart recorder 15.

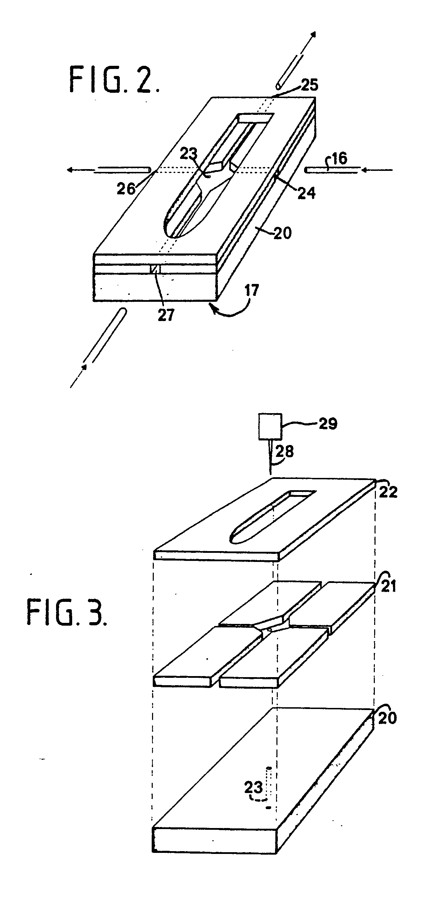

A tube 16 which is of polyethylene which could alternatively be made of some other inert plastics material such as polytetrafluoroethylene has an external diameter of 1.00 mm and an internal diameter of 0.5 mm, is connected to outlet 6 from the blood container 1, and passes through a jig 17, which will be described in more detail hereafter with reference to Figures 2 and 3.

After passage through the jig 17, waste blood is passed into a closed drainage vessel 18. Vessel 18 has a tube 19 connected to its outlet which dips into a pool of mercury. Adjustment of the height of the mercury column in the tube 19 thus provides a readily measurable and variable back-pressure to the blood in the tube 16.

Figures 2 and 3 illustrate in more detail the jig and tube-piercing arrangement used in the apparatus of Figure 1. A jig for enabling an accurate through hole to be made in the polyethylene tubing 16. In Figures 2 and 3, a support for the tubing 16 includes a lower support block 20 of polymethylmethacrylate, with small hole 23, having a diameter of approximately 0.15 mm provided therein. Four polymethylmethacrylate spacer blocks 21 are secured to the support block 20 with adhesive, and define between them four channels, 24, 25, 26, and 27. The jig assembly is completed by a cover plate 22. The polyethylene tubing 16 is passed through the channels 24 and 26 defined by the spacer blocks 21, so that it covers the hole 23 (tube 16 is shown broken in Figure 2 for clarity). A saline solution at a constant temperature of 37°C is supplied to inlet 27, and a suction supply to passageway 25, to remove the saline, and blood washed from the tube 16, to permit observation. A stainless steel needle 28 having a parallel-sided shank, 0.14 mm in diameter, and a length of approximately 15 mm is affixed to a needle-support 29, which is mounted on a slide (not shown) for movement towards and away from the hole 23, substantially normally to the block 20. Thus, the polyethylene tubing 16 can be manipulated within the block 17 so that the needle 28 enters the tube substantially perpendicular and pierces a through hole in it, i.e. pierces the tube 16 such that the parallel-sided portion of the shank passes completely through the tube, so as to produce in both walls of the tube a hole having a size which is completely reproducible.

In practice, the needle holder 29 is fitted onto the adjustable lens holder of an inverted microscope (WILD). This arrangement has the advantage of not disturbing the illumination pathway. The microscope is illustrated schematically in Figure 1. The position of the needle 28 with respect to the tubing 16 is observed by means of a closed-circuit television system, illustrated schematically at 30 in Figure 1, coupled to the microscope. Because of the optical system great precision can be obtained in the positioning of the holes perpendicular to the tubing.

In use of the apparatus, the syringe 12 is filled with light paraffin oil, and the container 1 with anticoagulated blood (for example buffered with heparain, citrate, or EDTA). A new tube 16 is applied to the outlet 6 of tube 1, and cause to pass through jig 17, and into vessel 18. A layer of hydrocarbon oil is floated on the surface of blood, using branch 10 of the Y tube 9, and branch 10 is then sealed.

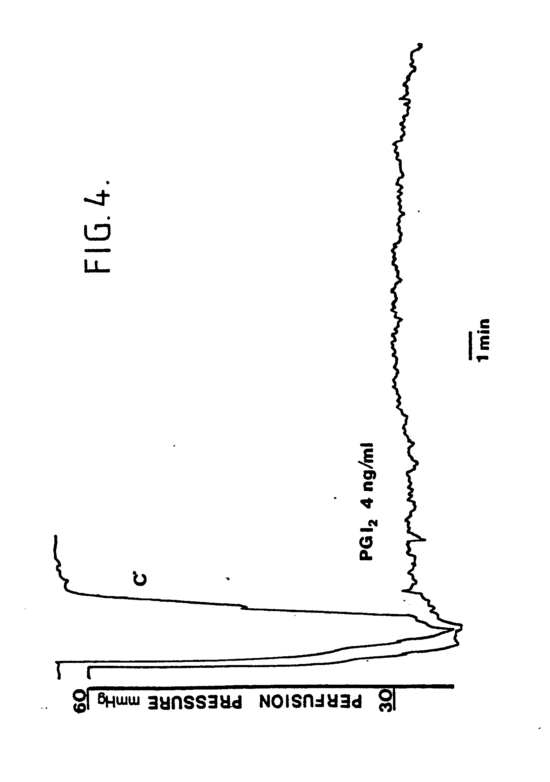

Operation of the syringe pump 13 is then commenced, until a steady flow of stirred blood is obtained in tube 16. The back-pressure is adjusted to 60 mm Hg, by varying the height of the mercury column in tube 19. The pressure in the blood vessel 1 is meanwhile monitored by means of pressure transducer 14, and the blood is maintained at a constant 37°C by means of waterjacket 2. When the pressure reading on chart recorder 15 indicates that a steady state has been reached, needle 28 is passed through the tube 16, and into hole 23, so as to begin the "bleeding". The buildup of a platelet "plug" is meanwhile observed by tracing the pressure rise, using the microscope and television system. Any water flowing through the tube 19 may be sucked off from the mercury surface by suction apparatus. Figure 4 shows a trace of the pressure indicated by the chart recorder 15 for two samples, a control sample of normal blood, (indicated as C on Figure 4) and a sample containing prostacyclin added to the blood (PGI$_2$ on Figure 4). It can be seen that the sample to which prostacyclin has been added shows a substantially increased bleeding time.

The pumping technique utilising displacement by hydrocarbon oil has been found to cause no appreciable damage to the red cells of previous techniques have been found to cause considerable damage, and I have even found that a number of other fluids which might have been expected to be inert and therefore as effective as hydrocarbon oil in fact cause considerable damage. For example, silicone fluid has been found to cause some damage to red cells.

The technique allows described above is suitable for determination of the haemostasis reaction of *non-anticoagulated blood* that is to say, blood to which no anti-coagulant has been added, provided that the blood sample is used immediately after being taken. The technique indicates that haemostasis is generally completed in from 3 to 5 minutes.

When haemostasis is complete, a steady perfusion pressure baseline is obtained until the blood clots. That moment is sharply indicated by the sudden increase of the pressure.

Thus, information regarding both the haemostatic activity and the coagulation of the blood can be obtained from the same, non-anticoagulated blood sample.

The apparatus according to the invention may also be utilised for the measurement of thrombolysis of the blood, i.e., the time taken for thrombus to be dissolved. Such measurements are particularly useful when it is desired to assess the reaction of patients to thrombolytic agents, for example streptokinase, or urokinase. These agents are used clinically, to treat arterial thrombosis, and the amount of the agents needed for a particular patient is found to vary widely, depending on a number of factors, in particular the concentration in the blood of certain antibodies. In patients of this kind, it is essential that estimates be made as rapidly as possible of the amount of thrombolytic agents required.

By utilising in the method of the invention anticoagulated (e.g., heparin containing) blood, to which a thrombolytic agent such as steptokinase or urokinase has been added, it is possible to form an estimation of the time taken for thrombolysis to occur. In practice, this is done by causing the blood flow in the tube to cease when haemostasis occurs, for example by stopping the syringe pump motor 13. The way in which the pressure in the tube (16) decreases with time is then observed.

According to a further aspect of the invention, there is therefore provided a method of measuring thrombolytic activity of an anticoagulated blood sample, which method comprises causing the blood to flow through a tube under substantially constant pressure, piercing a hole in the tube whilst the blood is flowing in it, causing blood flow in the tube to cease when haemostasis occurs, and observing the decrease in pressure in the tube with time, to determine the thrombolytic activity of the blood. The pressure will in general remain steady when the initial haemostatic plug has formed, but will decrease as the thrombosis dissolves, at a rate proportional to the leakage through the holes.

In this way, thrombolysis can be tested using only a very small volume of blood.

A similar method can be employed without the addition of thrombolytic agents, to determine the spontaneous thrombolytic activity of a blood sample. Such measurement can be useful in the investigation of clinical conditions such as disseminated intravascular coagulation syndrome.

It will of course be readily understood that a large number of variations of the above-described specific embodiments are possible, within the scope of the present invention as claimed.

**Claims**

1. Apparatus for determining the haemostatic reaction of a blood sample, which apparatus comprises a container (1) for the blood sample, means (12, 13) for causing the blood sample to flow through a tube (16), a support for the tube and means for measuring the outflow of blood from the tube, characterised in that the apparatus comprises a needle (28) having a substantially parallel sided shank mounted for movement to pierce a through hole in both walls of the tube (16) substantially perpendicular to its surface, whilst the blood is flowing in the tube.

2. Apparatus as claimed in Claim 1 which apparatus includes means (18) for controlling the back-pressure applied to the blood in the tube, to enable the said back-pressure to be maintained at substantially the arterial pressure.

3. Apparatus as claimed in Claim 1 or Claim 2 comprising a support jig (17) adapted to support the tube in a fixed position, and including means for supporting the needle (28) for movement towards and away from the tube.

4. Apparatus as claimed in Claim 3, wherein a hole (23) having a size such as to be a clearance fit for the needle (28) is provided in a surface of the jig adapted to support the tube.

5. Apparatus as claimed in any one of the preceding claims, including means (30) for viewing the tube under magnification.

6. Apparatus as claimed in any one of the preceding claims, including means (2) for maintaining the blood sample at a substantially constant temperature.

7. Apparatus as claimed in any one of the preceding Claims, wherein the container (1) for the blood sample is a columnar container, and has an outlet (6) for the blood at the lower end thereof, and wherein means (12, 13) are provided for, causing a displacing fluid, to pass into the lower end of the columnar container (1) to displace the blood through the outlet (6).

8. Apparatus as claimed in Claim 7, wherein the columnar container (1) is such that droplets of the displacing fluid formed in the column are substantial in size in comparison with the diameter of the columnar container (1).

9. A method of determining the haemostatic reaction of a blood sample, which method comprises causing the blood to flow through the tube (16) of apparatus as claimed in any one of the preceding Claims, under substantially constant pressure, piercing a through hole in both walls of the tube whilst the bood is flowing in it with the substantially parallel-sided needle, and measuring the time necessary for blood flow through the holes substantially to cease.

10. A method of measuring the thrombolytic activity of an anticoagulated blood sample, which method comprises causing the blood to flow through the tube of apparatus as claimed in any one of Claims 1 to 8 under substantially constant pressure, piercing a hole in the tube whilst the blood is flowing in it using the substantially parallel-sided needle, causing blood flow in the tube to cease when haemostasis occurs, and observing the decrease in pressure in the tube with time, to determine the thrombolytic activity of the blood.

**Patentansprüche**

1. Vorrichtung zur Bestimmung der Blutgerinnungszeit einer Blutprobe, wobei die Vorrichtung einen Behälter (1) für die Blutprobe, eine Einrichtung (12, 13), um zu bewirken, daß die Blutprobe durch ein Rohr (16) fließt, einen Halter für das Rohr und eine Einrichtung zun Merssen des Blutablaufs aus dem Rohr, umfaßt, dadurch gekennzeichnet, daß die Vorrichtung eine Nadel (28) mit einem Schaft mit im wesentlichen parallelen Seiten umfaßt, die vorgesehen ist für eine Bewegung, um ein Durchgangsloch in· beide Wände des Rohres (16) im wesentlichen senkrecht zu dessen Oberfläche zu stechen, während das Blut in dem Rohr fließt.

2. Vorrichtung nach Anspruch 1, wobei die Vorrichtung eine Einrichtung (18) zum Regeln des Rückstaus umfaßt, der auf das Blut in dem Rohr aufgebracht wird, um zu ermöglichen, daß der Rückstau im wesentlichen ʻauf dem arteriellen Druck gehalten wird.

3. Vorrichtung nach Anspruch 1 oder 2, mit einer Aufspannvorrichtung (17) zum Halten des Rohres in einer festen Position und mit einer Einrichtung zum Halten der Nadel (28) für eine Bewegung zu dem Rohr hin und von dem Rohr weg.

4. Vorrichtung nach Anspruch 3, wobei ein Loch (23) eine Größe derart aufweist, daß eine Spielpassung für die Nadel (28) in einer Fläche der Aufspannvorrichtung vorgesehen ist, die zum Halten des Rohres ausgebildet ist.

5. Vorrichtung nach einem der vorhergehenden Ansprüche, mit einer Einrichtung (30) zum Betrachten des Rohres unter Vergrößerung.

6. Vorrichtung nach einem der vorhergehenden Ansprüche, mit einer Einrichtung (2) zum Halten der Blutprobe auf im wesentlichen konstanter Temperatur.

7. Vorrichtung nach einem der vorhergenden Ansprüche, wobei der Behälter (1) für die Blutprobe ein säulenförmiger Behälter ist und einen Auslaß (6) für das Blut am unteren Ende aufweist und wobei eine Einrichtung (12, 13) vorgesehen ist, mit welcher bewirkt wird, daß ein Verdrängungsfluid in das untere Ende des säulenartigen Behälters (1) gelangt, um das Blut durch den Auslaß (6) zu verdrängen.

8. Vorrichtung nach Anspruch 7, wobei der säulenartige Behälter (1) derart ausgebildet ist, daß Tröpfchen des Verdrängerfluids, die in der Säule gebildet werden, in der Größe im wesentlichen vergleichbar sind mit dem Durchmesser des säulenartigen Behälters (1).

9. Verfahren zur Bestimmung der Blutgerinnungszeit einer Blutprobe, wobei das Verfahren umfaßt, daß das Blut durch das Rohr (16) der Vorrichtung nach einem der vorhergenden Ansprüche unter im wesentlichen konstantem Druck fließt, daß ein Durchgangsloch in beide Wände des Rohres mit der Nadel mit im wesentlichen parallelen Seiten gestochen wird, während das Blut darin fließt, und daß die Zeit gemessen wird, die erforderlich ist, damit der Blutfluß durch die Löcher im wesentlichen aufhört.

10. Verfahren zur Messung der Thrombinaktivität einer antikoagulierten Blutprobe, wobei das Verfahren umfaßt, daß bewirkt wird, daß das Blut durch das Rohr der Vorrichtung nach einer der Ansprüche 1 bis 8 bei im wesentlichen konstantem Druck fließt, daß ein Loch in das Rohr unter Verwendung der Nadel mit im wesentlichen parallelen Seiten gestochen wird, während das Blut darin fließt, daß bewirkt wird, daß der Blutfluß in dem Rohr beendet wird, wenn eine Blutgerinnung auftritt, und daß der Druckabfall in dem Rohr über die Zeit beobachtet wird, um die Thrombinaktivität des Blutes zu bestimmen.

**Revendications**

1. Appareil servant à déterminer la réaction d'hémostase d'un échantillon de sang, comprenant un récipient (1) pour l'échantillon de sang, des moyens (12, 13) pour produire l'écoulement de l'échantillon de sang à travers un tube (16), un support pour le tube et des moyens pour mesurer le débit de sortie du sang à partir du tube, caractérisé en ce que l'appareil comprend une aiguille (28) comportant une tige à faces sensiblement parallèles, montée mobile de façon à percer un trou traversant dans les deux parois du tube (16) dans une direction pratiquement perpendiculaire à la surface de celui-ci, tandis que le sang coule dans le tube.

2. Appareil selon la revendication 1, comprenant des moyens (18) pour contrôler la contre-pression appliquée au sang dans le tube, afin de permettre que cette contre-pression soit maintenue sensiblement à la pression artérielle.

3. Appareil selon la revendication 1 ou 2, comprenant une monture de support (17) agencée de façon à supporter le tube en position fixe et comprenant des moyens de montage de l'aiguille (28) permettant le mouvement de celle-ci vers et à partir du tube.

4. Appareil selon la revendication 3, dans lequel un trou (23) dimensionné pour un ajustement avec jeu de l'aiguille (28) est pratiqué dans la surface de la monture destinée à supporter le tube.

5. Appareil selon l'une quelconque des revendications 1 à 4, comprenant des moyens (30) pour observer le tube avec grossissement.

6. Appareil selon l'une quelconque des revendications 1 à 5, comprenant des moyens (2) pour maintenir l'échantillon de sang à une température sensiblement constante.

7. Appareil selon l'une quelconque des revendications 1 à 6, dans lequel le récipient (1) pour l'échantillon de sang est un récipient en forme de colonne et comporte un orifice de sortie (6) pour le sang à son extrémité inférieure, et dans lequel des moyens (12, 13) sont prévus pour faire pénétrer un fluide de déplacement dans l'extrémité inférieure du récipient en forme de colonne (1) afin de déplacer le sang à travers l'orifice de sortie (6).

8. Appareil selon la revendication 7, dans lequel le récipient en forme de colonne (1) est tel que les gouttelettes du fluide de déplacement formées dans la colonne aient une taille importante en comparaison du diamètre du récipient en forme de colonne (1).

9. Procédé de détermination de la réaction d'hémostase d'un échantillon de sang, consistant à produire l'écoulement du sang, sous une pression sensiblement constante, à travers le tube (16) de l'appareil selon l'une quelconque des revendications 1 à 8, à percer un trou traversant dans les deux parois du tube, tandis que le sang coule dans celui-ci, avec l'aiguille à faces sensiblement parallèles, et à mesurer le temps nécessaire pour que l'écoulement de sang à travers les trous cesse pratiquement.

10. Procédé de mesure de l'activité thrombolytique d'un échantillon de sang traité par un anticoagulant, consistant à produire l'écoulement du sang, sous une pression sensiblement constante, à travers le tube de l'appareil selon l'une quelconque des revendications 1 à 8, à percer un trou dans le tube, tandis que le sang coule dans celui-ci, au moyen de l'aiguille à faces sensiblement parallèles, à faire en sorte que l'écoulement de sang dans le tube cesse lorsque l'hémostase se produit, et à observer la baisse de pression dans le tube avec le temps, pour déterminer l'activité thrombolytique du sang.

FIG.1.

FIG. 2.

FIG. 3.

FIG. 4.